# EUROPÄISCHE PATENTSCHRIFT

(11) **EP 1 222 242 B1**
(45) Veröffentlichungstag und Bekanntmachung des Hinweises auf die Patenterteilung: **20.04.2005**
(21) Anmeldenummer: 00971337.1
(22) Anmeldetag: 07.10.2000
(51) Int. Cl.: C11D 3/02, C11D 3/382, C11D 3/34, C11D 3/33, C11D 3/16, A61K 7/48, A61K 7/06

(54) **TENSIDHALTIGE REINIGUNGSMITTEL MIT ENZYM-INHIBITOREN**
CLEANING AGENTS WHICH CONTAIN SURFACTANTS AND ENZYME INHIBITORS
NETTOYANTS TENSIOACTIFS COMPORTANT DES INHIBITEURS D'ENZYMES

(30) Priorität: 16.10.1999 DE 19950019
(43) Veröffentlichungstag der Anmeldung: 17.07.2002
(73) Patentinhaber: Henkel Kommanditgesellschaft auf Aktien, 40589 Düsseldorf (DE)
(72) Erfinder: SÄTTLER, Andrea, 40225 Düsseldorf (DE); FÖRSTER, Thomas, D-40699 Erkrath (DE); SCHLOTMANN, Kordula, 40225 Düsseldorf (DE); WEISS, Albrecht, 40764 Langenfeld (DE)
(86) Internationale Anmeldenummer: PCT/EP2000/009832
(87) Internationale Veröffentlichungsnummer: WO 2001/029163

(56) Entgegenhaltungen:
- EP-A- 0 478 050
- WO-A-94/29428
- WO-A-96/01101
- DE-A- 2 060 485
- DE-A- 3 741 617
- FR-A- 2 208 643
- GB-A- 1 224 564
- GB-A- 1 236 709
- GB-A- 1 513 865
- US-A- 5 322 839
- US-A- 5 393 526

## Beschreibung

Die Erfindung betrifft tensidhaltige Zusammensetzungen, die als bioaktive Komponenten Enzyminhibitoren enthalten.

Reinigungsmittel, beispielsweise Körperreinigungsprodukte und insbesondere Haushaltsreiniger, führen bei häufiger Anwendung oft zu mehr oder minder starker Hautaustrocknung und -entfettung mit verstärkter Abschuppung. Um eine hautschonende Reinigung zu ermöglichen, werden zum einen hautfreundliche Tenside eingesetzt und vielen Reinigungsmitteln auch sogenannte Rückfetter zugesetzt.

Ein besonderes Anforderungsprofil ergibt sich für Reinigungsmittel, die auch bei trockener und zu Austrockunung neigender Haut verwendet werden können. Die Entstehung trockener Haut wurde intensiv untersucht (K. Kitamura, K. Yamada, A. Ito und M. Fukuda *J. Soc. Cosm. Chem. Japan* **1995**, *29 (2)*, 133-145), die zugrundeliegenden biochemischen Prozesse sind jedoch bislang nicht völlig geklärt.

Gesunde Haut wird durch ein Fließgleichgewicht, welches die mittlere Schichtdicke der Haut aufrecht erhält, ständig erneuert. Die Zellen der Hornschicht (Stratum corneum) an der Hautoberfläche werden abgeschuppt, während aus dem Stratum basale neue Zellen nachgeliefert werden. Für den Abschuppungsprozeß (Desquamation) müssen die Proteinstrukturen (Desmosomen), die für die Kohäsion der Zellen verantwortlich sind, aufgelöst werden (A. Lundström, T. Egelrud, *J. Invest. Dermatol.* **1988,** *91,* 340-343 und **1990,** *94,* 216-220; idem, *Arch. Dermatol. Res.* **1990,** *282,* 234-237). Der Abbau der Desmosomen erfolgt über im Stratum comeum enthaltene Proteasen, von denen zwei näher charakterisiert wurden. Es handelt sich hierbei um Serin-Proteasen vom Chymotrypsin- und Trypsin-Typ (T. Egelrud et al., *Acta Derm. Venereol.* **1991**, *71*, 471-474; JP 8068791 A2).

Trockene Haut zeichnet sich durch eine verstärkte Abschuppung von Keratinozyten in der Epidermis aus.

Die derzeit eingesetzten Reinigungsprodukte können die Hautaustrocknung noch nicht zufriedenstellend beeinflussen. Aufgabe war es, Reinigungsprodukte zu entwickeln, welche die Abschuppung der Keratinozyten durch gezielt wirkende bioaktive Komponenten reduzieren, indem sie die am Desmosomenabbau beteiligten Proteasen hemmen.

Überraschenderweise wurde nun eine Gruppe von Inhibitoren gefunden, welche die an der Abschuppungsreaktion der Haut beteiligten Proteasen hemmen und die Keratinozyten länger im Zellverbund halten. Als bioaktive Komponenten in Reinigungsmitteln reduzieren sie den insbesondere bei trockener Haut abnorm verstärkten Abschuppungsprozeß und normalisieren so das Erscheinungsbild der Haut, die den tensidhaltigen Mitteln ausgesetzt ist. Ebenso wird bei gereizter und aus diesem Grund stark abschuppender Haut ein Hautschutz erreicht.

Gegenstand der Erfindung ist daher ein tensidhaltiges Reinigungsmittel gemäß Anspruch 1. Gegenstand der Erfindung ist die Verwendung des erfindungsgemäßen Mittels zur topisch prophylaktischen und/oder kosmetischen Behandlung trockener Haut gemäß Anspruch 5. Gegenstand der Erfindung ist weiterhin ein Verfahren zur manuellen Reinigung harter Oberflächen mit tensidhaltigen Reinigungsmitteln bei gleichzeitiger Verhinderung oder Verminderung der Hautaustrocknung gemäß Anspruch 7.

Wasserlösliche Inhibitoren mit einem Molekulargewicht kleiner als 5000 g/mol können im Sinne der Erfindung bevorzugt sein, wobei unter Wasserlöslichkeit eine minimale Löslichkeit des Inhibitors von 0,001 Gew.-% in Wasser bei 25°C verstanden wird. Unter Inhibition oder Hemmung im Sinne der Erfindung wird die Aktivitätsabnahme (Definition der Enzymaktivität, vgl. Stichwort "Enzyme" in: CD Römpp Chemie Lexikon, Version 1.0, Stuttgart, New York: Georg Thieme Verlag 1995) des entsprechenden, aus der Haut isolierten Enzyms oder eines geeigneten Modellenzyms in Anwesenheit des Inhibitors oder eines Gemisches von Inhibitoren im *in-vitro*-Test und/oder die verminderte Abschuppung von Keratinozyten an Hautbiopsien in Anwesenheit des Inhibitors oder eines Gemisches von Inhibitoren verstanden. Der Inhibitor oder das Gemisch von Inhibitoren ist vorzugsweise in einer Konzentration von 0,0001 - 20 Gew.-% und insbesondere 0,1 - 5 Gew.-% in den Zusammensetzungen enthalten. Als tensidhaltig werden im Sinne der Erfindung Mittel bezeichnet, die wenigstens 5 Gew.-% eines Tensids oder Tensidgemisches enthalten. Zu den erfindungsgemäßen Mitteln zählen u.a. Badezusätze, Duschpräparate, Shampoos, Spülmittel, Seifen und Haushaltsreiniger. Je nach Zweckbestimmung können die tensidhaltigen Lösungen neben den üblichen anionischen, kationischen, nichtionischen und amphoteren Tensiden u.a. konsistenzgebende Komponenten, Konditionierungsmittel und pflegende Wirkstoffe enthalten.

Als Inhibitoren sind Verbindungen enthaltend die Pentapeptidsequenz Glycin-Prolin-Phenylalanin-Prolin-Leucin, erfindungsgemäß, da sie sich als besonders effektiv erwiesen haben.

Im Sinne der Erfindung bevorzugt sind Inhibitoren, die die am Desmosomen-Abbau beteiligten Serin-Proteinasen hemmen. Diese Proteinasen enthalten im aktiven Zentrum einen für die Katalyse essentiellen L-Serin-Rest. Als Inhibitoren im Sinne der Erfindung eignen sich Substanzen, die den L-Serin-Rest modifizieren und/oder die Substratspaltungsstelle durch Wechselwirkung mit dem L-Serin-Rest bzw. mit Aminosäuren aus der Umgebung blockieren und/oder über die Änderung der Tertiärstruktur des Enzyms dessen Inaktivierung bewirken. Als Hemmwirkung im Sinne der Erfindung wird, wie bereits für den allgemeinen Fall erläutert, eine Aktivitätsabnahme der Serin-Proteinasen in Anwe senheit des Inhibitors im *in-vitro*-Test und/oder die verminderte Abschuppung von Keratinozyten an Hautbiopsien in Anwesenheit des Inhibitors bezeichnet.

Besonders bevorzugt im Sinne der Erfindung ist ein Inhibitor, der die am Desmosomen-Abbau beteiligten Proteinasen vom Typ der Trypsin- oder Chymotrypsin-ähnlichen Serinproteinasen hemmt. Trypsin ist eine Endopeptidase, die im Dünndarm aus der vom Pankreas gebildeten Vorstufe Trypsinogen durch Abspaltung eines Hexapeptids entsteht. Kennzeichnend für diese Serin-Proteinase ist ein negativ geladener L-Aspartat-Rest in der Substrat-Bindungstasche, der die Spezifität des Enzyms mitbestimmt. Trypsin spaltet Peptidketten spezifisch auf der Carboxy-Seite der basischen Aminosäuren L-Lysin und L-Arginin. Chymotrypsin entsteht im Pankreas aus inaktiven Vorstufen, sogenannten Zymogenen, durch Einwirkung von Trypsin. Chymotrypsin spaltet Proteine, Peptide, Aminosäureester und Amide spezifisch an der Carboxy-Gruppe hydrophober Aminosäuren. Beide Enzym-Typen sind am Abbau von Proteinstrukturen (Desmosomen) beteiligt, welche für die Kohäsion der Homzellen in der Haut verantwortlich sind. Als Hemmwirkung im Sinne der Erfindung wird, wie bereits für den allgemeinen Fall erläutert, eine Aktivitätsabnahme der Trypsin- oder Chymotrypsin-ähnlichen Serin-Proteinasen in Anwesenheit des Inhibitors im *in-vitro*-Test bezeichnet und/oder die verminderte Abschuppung von Keratinozyten an Hautbiopsien in Anwesenheit des Inhibitors bezeichnet.

Inhibitor ist das Pentapeptid Glycin-Prolin-Phenylalanin-Prolin-Leucin (GPFPL) oder chemische Derivate, die diese Pentapeptidsequenz enthalten. Das Pentapeptid ist als Inhibitor für Serin-Proteinasen bekannt. Beansprucht im Sinne der Erfindung sind auch endgruppengeschützte Derivate dieser Pentapeptidsequenz, Polypeptide, Proteine und andere chemische Derivate, die diese Pentapeptidsequenz enthalten, die eine inhibitorische Wirkung in den umseitig beschriebenen Tests aufweisen. Eine deutliche Hemmung von Chymotrypsin (27 %) wurde in *in-vitro-Tests* bei Verwendung von 0.33 Gew.-% N-CBZ-Gly-Pro-Phe-Pro-Leu (N-CBZ=N-Benzyloxycarbonyl) beobachtet (Beispiel 4). Bei Verwendung von 0,5 % N-CBZ-GPFPL kann die Desquamation der Haut um die Hälfte reduziert werden (Beispiel 11).

### Tenside

Die Inhibitor-Wirkstoffe können in alle für Reinigungsmittel übliche Rezepturen eingearbeitet werden. Da dem Fachmann eine Fülle solcher Rahmenrezepturen bekannt ist, werden diese hier nicht im Detail aufgeführt. Neben Wasser und den obligatorischen Tensiden sowie physiologisch geeigneten Lösungsmitteln können die Rezepturen u. a. auch pflegende Bestandteile (z. B. Öle, Wachse, Fette, rückfettende Substanzen), Verdickungsmittel sowie Farb- und Duftstoffe enthalten. Die Zusammensetzung kann je nach Anforderung nahezu wasserfrei, als wäßrige oder alkoholische Lösung, als Gel oder als tensidhaltige O/W- oder W/O-Emulsion formuliert werden. Für geeignete Formulierungs-Grundlagen sei an dieser Stelle auf die in der Kosmetik üblichen Rezepturen verwiesen (K. Schrader, *Grundlagen und Rezepturen der Kosmetika,* 2. Aufl., Hüthig Buch Verlag, Heidelberg 1989, Seite 709-722, 606-618).

Vorzugsweise enthalten die Zusammensetzungen schaumstarke wasserlösliche Tenside. Hierzu zählen beispielsweise Aniontenside oder eine Kombination von Aniontensiden mit Alkylpolyglycosiden.

### Aniontenside

Als anionische Tenside bevorzugt sind feste oder pulverförmige Alkylsulfate, Alkylethercarboxylate, Isethionate wie z. B. Acylisethionate, Alkylsulfosuccinate und die Monoester der Sulfosuccinate. Aber auch Alkylsulfonate, Alkylethersulfate und -sulfonate, Alkylsuccinate, Diester von Sulfosuccinaten, N-Acylsarkosinate und N-Acyltaurine mit linearen Alkyl- oder Acylgruppen (12 - 18 C-Atome) sind in den Mengen einsetzbar, die eine Tablettierung ohne Verkleben ermöglichen. Die Aniontenside werden üblicherweise in Form ihrer Alkali, Magnesium-, Ammonium- oder Alkanolammoniumsalze eingesetzt.

### Sulfate

Als Alk(en)ylsulfate werden die Alkali- und insbesondere die Natriumsalze der Schwefelsäurehalbester der C₁₂-C₁₈-Fettalkohole aus natürlichen Fetten, beispielsweise Kokosfettalkohol, Talgfettalkohol, Lauryl-, Myristyl-, Cetyl- und Stearylalkohol bevorzugt. Ebenso geeignet sind die Alkali- und Natriumsalze der Schwefelsäurehalbester der C₁₀-C₂₀-Oxoalkohole und die Halbester sekundärer Alkohole dieser Kettenlängen. Weiterhin bevorzugt sind Alk(en)ylsulfate der genannten Kettenlänge, welche einen synthetischen, auf petrochemischer Basis hergestellten geradkettigen Alkylrest enthalten, die ein analoges Abbauverhalten besitzen wie die adäquaten Verbindungen auf der Basis von fettchemischen Rohstoffen. Für eine intensive Schaumbildung bei physiologisch verträglichen Temperaturen sind die C₁₂-C₁₆-Alkylsulfate und insbesondere die C₁₂-C₁₄-Alkylsulfate bevorzugt. Auch 2-Alkylsulfate, welche beispielsweise gemäß den US-Patentschriften 3,234,258 oder 5,075,041 hergestellt werden, sind geeignete Aniontenside.

Exzellente Schaumeigenschaften zeigen Alkylethersulfate. Bevorzugt aus dieser Gruppe sind Schwefelsäuremonoester der mit 1 bis 6 Mol Ethylenoxid ethoxylierten geradkettigen oder verzweigten C₇₋₂₁-Alkohole, wie 2-Methyl-verzweigte C₉₋₁₁-Alkohole mit im Durchschnitt 3,5 Mol Ethylenoxid (EO) oder C₁₂₋₁₈-Fettalkohole mit 1 bis 4 EO. Wegen ihres guten Schaumverhaltens können sie auch in relativ geringen Mengen eingesetzt werden.

Weitere geeignete Aniontenside vom Sulfattyp sind sulfatierte Fettsäureglycerinester. Unter Fettsäureglycerinestern sind die Mono-, Di- und Triester sowie deren Gemische zu verstehen, wie sie bei der Herstellung durch Veresterung von einem Mol Glycerin mit 1 bis 3 Mol Fettsäure oder bei der Umesterung von Triglyceriden mit 0,3 bis 2 Mol Glycerin erhalten werden. Bevorzugte sulfatierte Fettsäureglycerinester sind die Ester gesättigter Fettsäuren mit 6 bis 22 Kohlenstoffatomen, beispielsweise der Capronsäure, Caprylsäure, Caprinsäure, Myristinsäure, Laurinsäure, Palmitinsäure, Stearinsäure oder Behensäure.

Ferner können ethoxylierte Alkylphenolethersulfate, C₅-C₁₇-Acyl-N-(C₁-C₄)-alkylglucaminsulfate und -N-(C1-C2-hydroxyalkyl)glucaminsulfate sowie Sulfate von Alkylpolyglucosiden als Anionentenside eingesetzt werden.

### Sulfonate

Als Tenside vom Sulfonat-Typ kommen vozugsweise C₉₋₁₃-Alkylbenzolsulfonate und Olefinsulfonate in Betracht. Olefinsulfonate sind Gemische aus Alken- und Hydroxyalkansulfonaten sowie Disulfonaten, wie man sie beispielsweise aus C₁₂₋₁₈-Monoolefinen mit end- oder innenständiger Doppelbindung durch Sulfonieren mit gasförmigem Schwefeltrioxid und anschließende alkalische oder saure Hydrolyse der Sulfonierungsprodukte erhält. Geeignet sind auch Alkansulfonate, die aus C₁₂₋₁₈-Alkanen beispielsweise durch Sulfochlorierung oder Sulfoxidation mit anschließender Hydrolyse bzw. Neutralisation gewonnen werden. Ebenso sind auch die Ester von α-Sulfofettsäuren (Estersulfonate), z. B. die α-sulfonierten Methylester der hydrierten Kokos-, Palmkem- oder Talgfettsäuren geeignet.

Weitere geeignete, besonders schaumstarke Anionentenside des Sulfonat-Typs sind die Salze, insbesondere die Alkalimetallsalze, der Alk(en)ylsulfobernsteinsäure, die auch als Sulfosuccinate oder als Sulfobernsteinsäureester bezeichnet werden. Hierzu zählen die Mono- und/oder Diester der Sulfobernsteinsäure mit Alkoholen, vorzugsweise Fettalkoholen und insbesondere ethoxylierten Fettalkoholen. Die ethoxylierten Fettalkohole können eine normale oder eingeengte Homologenverteilung aufweisen. Bevorzugte Sulfosuccinate enthalten C₈₋₁₈-Fettalkoholreste oder Mischungen aus diesen. Vorzugsweise enthalten die Sulfosuccinate einen ethoxylierten Fettalkoholrest.

### Niotenside

Zur Gruppe der Niotenside gehören vor allem alkoxylierte Alkohole, alkoxylierte Fettsäureester, Alkylpolyglucoside und Aminoxide. Als Schaumstabilisatoren sind ferner Fettsäurealkanolamide und Fettsäurealkanolamidethoxylate einsetzbar.

### Alkoxylierte Fettalkohole

Alkoxylierte Fettalkohole haben je nach Alkoxylierungsgard und Länge des Alkylrestes unterschiedliche anwendungstechnische Eigenschaften. Sie werden u. a. als Emulgatoren und Lösungsvermittler für Fette und Öle in Dusch- und Schaumbädern eingesetzt. Vorteilhafterweise werden ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt. Der Alkylrest kann linear oder bevorzugt in 2-Stellung methylverzweigt sein. Auch Gemische alkoxylierter Alkohole mit linearen und methylverzweigten Resten, wie sie üblicherweise bei Oxoalkoholen auftreten, können eingesetzt werden. Bevorzugt sind Alkoholethoxylate mit 2 - 8 EO und linearen C₁₂-C₁₈-Alkylresten aus Alkoholen nativen Ursprungs, die beispielsweise aus Kokos-, Palm-, Talgfett- oder Oliven- oder Sonnenblumenöl gewonnen werden. Besonders bevorzugt sind die C₁₂₋₁₄-Alkohole mit 3 EO oder 4 EO, C₉₋₁₁-Alkohol mit 7 EO, C₁₃₋₁₅-Alkohole mit 3 EO, 5 EO, 7 EO oder 8 EO, C₁₂₋₁₈-Alkohole mit 3 EO, 5 EO oder 7 EO und Mischungen aus diesen, wie Mischungen aus C₁₂₋₁₄-Alkohol mit 3 EO und C₁₂₋₁₈-Alkohol mit 5 EO. Die angegebenen Ethoxylierungsgrade stellen statistische Mittelwerte dar, die für ein spezielles Produkt eine ganze oder eine gebrochene Zahl sein können. Auch Alkoholethoxylate mit eingeengter Homologenverteilung (narrow range ethoxylates, NRE) sind einsetzbar. Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO.

### Alkylpolyglycoside

Außerdem können als weitere nichtionische Tenside auch Alkylglykoside eingesetzt werden, die aus Zuckern und Alkoholen unter Acetalisierung hergestellt werden. Diese Tenside sind besonders gut hautverträglich und entfalten in Verbindung mit Aniontensiden besonders feinblasige und stabile Schäume.

Im Sinne der Erfindung sind wasserfreie Zuckertenside, wie sie in DE 43 40 015 und DE 44 04 633 beschrieben sind, bevorzugt. Auch sprühbare, wasserfreie APG/Niotensid-Mischungen, wie sie in DE 198 58 923.3 beschrieben sind, können vorteilhaft sein.

Als Zuckerkomponenten der Alkylglykoside (Glycosen) kommen bevorzugt Glucose, daneben aber auch Fructose, Mannose, Galactose, Talose, Gulose, Allose, Idose, Arabinose, Xylose, Lyxose, Ribose und Gemische davon in Frage. Bevorzugt wegen der leichten Verfügbarkeit und der guten Anwendungseigenschaften sind die Acetalisierungsprodukte der Glucose mit Fettalkoholen, die z. B. aus natürlichen Ölen und Fetten nach bekannten Verfahren erhältlich sind. Aber auch Polysaccharide, wie z. B. Stärke und Maltodextrin und die aus technischen Alkoholsynthesen zugänglichen Oxo- und Ziegleralkohole sind geeignete Ausgangsstoffe.

Bezüglich des Glycosidrestes gilt, daß sowohl Monoglycoside als auch Oligoglycoside, bei denen ein Zuckerrest glycosidisch an den Fettalkohol gebunden ist, geeignet sind. Gewöhnlich liegen bei den Handelsprodukten Gemische von Mono- und Oligoglycosiden vor, die ihrerseits Gemische verschiedener isomerer Formen darstellen.

Bevorzugt sind Alkylglykoside der Formel R¹O(G)ₓ, worin R¹ ein primärer geradkettiger oder methylverzweigter, insbesondere in 2-Stellung methylverzweigter aliphatischer Rest mit 8 bis 22, vorzugsweise mit 12 bis 18 C-Atomen ist, und G eine Glykoseeinheit mit 5 oder 6 C-Atomen, vorzugsweise Glucose, repräsentiert. Der Oligomerisierungsgrad x, der die Verteilung von Monoglykosiden und Oligoglykosiden angibt, ist eine beliebige Zahl zwischen 1 und 10; vorzugsweise liegt x bei 1 - 2, insbesondere bei 1,1 - 1,4.

Bevorzugt eingesetzt werden lineare Alkylpolyglucoside, also Alkylglycoside auf der Basis von Glucose und einem n-Alkylrest.

### Alkoxylierte Fettsäuren und Fettsäureester

Eine weitere Klasse nichtionischer, erfindungsgemäß verwendbarer Tenside sind alkoxylierte, vorzugsweise ethoxylierte oder ethoxylierte/propoxylierte Fettsäureester, vorzugsweise von Alkoholen mit 1 bis 4 C-Atomen oder Glycerin, beispielsweise alkoxylierte Fettsäuremethylester, die in der japanischen Patentanmeldung JP 58217598 beschrieben sind oder die nach dem in der internationalen Patentanmeldung WO-A-90/13533 beschriebenen Verfahren hergestellt werden.

### Aminoxide

Aminoxide werden durch Umsetzung von tertiären Aminen mit Wasserstoffperoxid gewonnen. Es handelt sich um gut wasserlösliche, biologisch abbaubare Tenside, die wegen ihrer milden Reinigungswirkung, ihrer hervorragenden Hautverträglichkeit, ihrer Schaumstabilität und geringen Toxizität für Badezusätze besonders geeignet sind. Hierzu gehören beispielsweise N-Kokosalkyl-N,N-dimethylaminoxid und N-Talgalkyl-N,N-dihydroxyethylaminoxid.

### Polyhydroxyfettsäureamide

Auch Polyhydroxyfettsäureamide weisen insbesondere in Kombination mit Aniontensiden eine gute Schaumstabilität auf. Sie sind biologisch abbaubar und sehr gut hautverträglich.

Geeignet sind Polyhydroxyfettsäureamide der Formel R²CONR³[Z], in der R²CO für einen aliphatischen Acylrest mit 6 bis 22 Kohlenstoffatomen, R³ für Wasserstoff, einen Alkyl- oder Hydroxyalkylrest mit 1 bis 4 Kohlenstoffatomen und [Z] für einen linearen oder verzweigten Polyhydroxyalkylrest mit 3 bis 10 Kohlenstoffatomen und 3 bis 10 Hydroxylgruppen steht. Bei den Polyhydroxyfettsäureamiden handelt es sich um bekannte Stoffe, die üblicherweise durch reduktive Aminierung eines reduzierenden Zuckers mit Ammoniak, einem Alkylamin oder einem Alkanolamin und nachfolgende Acylierung mit einer Fettsäure, einem Fettsäurealkylester oder einem Fettsäurechlorid erhalten werden können.

Zur Gruppe der Polyhydroxyfettsäureamide gehören auch Verbindungen der Formel 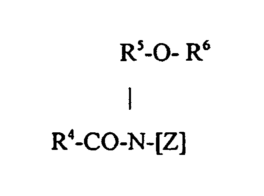 in der R⁴ für einen linearen oder verzweigten Alkyl- oder Alkenylrest mit 7 bis 12 Kohlenstoffatomen, R⁵ für einen linearen, verzweigten oder cyclischen Alkylrest oder einen Arylrest mit 2 bis 8 Kohlenstoffatomen und R⁶ für einen linearen, verzweigten oder cyclischen Alkylenrest oder einen Arylenrest oder einen Oxy-Alkylrest mit 1 bis 8 Kohlenstoffatomen steht, wobei C₁₋₄-Alkyl- oder Phenylreste bevorzugt sind. [Z] steht für einen linearen Polyhydroxyalkylrest, dessen Alkylkette mit mindestens zwei Hydroxylgruppen substituiert ist, oder alkoxylierte, vorzugsweise ethoxylierte oder propoxylierte Derivate dieses Restes.

[Z] wird vorzugsweise durch reduktive Aminierung eines reduzierten Zuckers erhalten, beispielsweise Glucose, Fructose, Maltose, Lactose, Galactose, Mannose oder Xylose. Die N-Alkoxy- oder N-Aryloxy-substituierten Verbindungen können dann beispielweise entsprechend der internationalen Anmeldung WO-A-95/07331 durch Umsetzung mit Fettsäuremethylestem in Gegenwart eines Alkoxids als Katalysator in die gewünschten Polyhydroxyfettsäureamide überführt werden.

### Amphotenside

Auch Amphotenside können in den erfindungsgemäßen Zubereitungen enthalten sein. Neben einer guten Haut- und Schleimhautverträglichkeit besitzen sie ein gutes Schaumund Reinigungsvermögen und weisen z. T. mikrobizide Wirkung auf. Unter ampholytischen Tensiden werden solche oberflächenaktiven Verbindungen verstanden, die außer einer C₈-C₁₈-Alkyl- oder Acylgruppe im Molekül mindestens eine freie Aminogruppe und mindestens eine -COOH- oder ―SO₃H-Gruppe enthalten. Sie haben die Eigenschaft, in saurer Lösung durch Protonierung am tertiären Stickstoffatom wie Kationtenside und im alkalischen Bereich durch Salzbildung an der Carboxylgruppe wie anionische Tenside zu reagieren. Amphotenside können innere Salze ausbilden. Beispiele für geeignete Amphotenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils 8 bis 18 C-Atomen in der Alkylgruppe. Besonders bevorzugte Amphotenside sind das N-Kokosalkylaminopropionat, das Kokosacylaminoethylaminopropionat, C₁₂₋₁₈-Acylsarcosine und Cocoamphoglycinate. Zur letzten Gruppe gehört beispielsweise das unter dem Warenzeichen Dehyton®G im Handel erhältliche Produkt.

### Zwitterionische Tenside

Zwitterionische Tenside sind ebenfalls seit langem bekannt und werden in einer Vielzahl kosmetischer Rezepturen eingesetzt. Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Die bekannteste und am weitesten verbreitete Gruppe dieser Tenside ist die der Betain-Tenside, bei denen die quartäre Ammoniumgruppe zwei Methylsubstituenten trägt. Geeignete zwitterionische Betain-Tenside sind u. a. N-Alkyl-N,N-dimethylammoniumglycinate, beispielsweiseKokosalkyldimethylammoniumglycinate, N-Acyl-aminopropyl-N,N-dimethylammoniumglycinate, beispielsweise Kokosacylaminopropyldimethylammoniumglycinate, und 2-niumglycinate, und 2-Alkyl-3-carboxylmethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Besonders geeignet sind Kokosacylaminopropyldimethylammoniumglycinate, die unter der INCI-Bezeichnung Cocamidopropylbetain bekannt sind, insbesondere Cocoamidopropylbetain, das von C₈-C₁₈-Kokos- oder Palmkernfettsäuren abgeleitet ist. Solche Produkte sind z. B. unter dem Warenzeichen Dehyton®K im Handel.

### Pflegekomponenten (fakultativ)

Die erfindungsgemäße tensidhaltige Zusammensetzung kann außerdem eine Reihe pflegender Komponenten enthalten. Im Sinne der Erfindung werden unter Pflegekomponenten allgemein Verbindungen verstanden, die einen pflegenden Einfluß auf die Haut ausüben. Hierzu zählen lipophile Verbindungen, wie beispielsweise Kohlenwasserstoffe, Silikonöle, höhere Alkohole und Fettsäuren sowie Fette, Öle und Wachse, die eine Rückfettung der Haut bewirken, oder kurativ oder systemisch wirkende Substanzen wie beispielsweise Ceramide, die einen Mangel an Homschichtlipiden auszugleichen vermögen. Auch Vitamine, Lipoproteine, Glycolipide, Phospholipide und Pflanzenextrakte mit dermatologischen Wirkstoffen sind als Pflegekomponenten einsetzbar.

### Natürliche und synthetische Öle und Fette

Hierzu zählen u.a. die Fettsäure- und Fettalkoholester, insbesondere die Monoester der Fettsäuren mit Alkoholen mit 1 bis 24 C-Atomen oder mit Glycerin. Bei dieser Stoffgruppe handelt es sich um die Produkte der Veresterung von Fettsäuren mit 8 bis 24 C-Atomen wie beispielsweise Capronsäure, Caprylsäure, 2-Ethylhexansäure, Caprinsäure, Laurinsäure, Isotridecansäure, Myristinsäure, Palmitinsäure, Palmoleinsäure, Stearinsäure, Isostearinsäure, Ölsäure, Elaidinsäure, Petroselinsäure, Linolsäure, Linolensäure, Elaeostearinsäure, Arachinsäure, Gadoleinsäure, Behensäure und Erucasäure sowie deren technische Mischungen, die z. B. bei der Druckspaltung von natürlichen Fetten und Ölen oder der Dimerisierung von ungesättigten Fettsäuren anfallen, mit Alkoholen wie beispielsweise Isopropylalkohol, Capronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalkohol, Petroselinylalkohol, Linolylalkohol, Linolenylalkohol, Elaeostearylalkohol, Arachylalkohol, Gadoleylalkohol, Behenylalkohol, Erucylalkohol und Brassidylalkohol sowie deren technische Mischungen, die z. B. bei der Hochdruckhydrierung von technischen Methylestern auf Basis von Fetten und Ölen oder von Aldehyden aus der Roelen'schen Oxosynthese sowie als Monomerfraktion bei der Dimerisierung von ungesättigten Fettalkoholen anfallen. Auch die hydrierten oder gehärteten Öle, z. B. hydriertes Sojaöl, Rizinusöl und Erdnußöl können verwendet werden.

Als Ölkörper geeignet sind auch Vaseline, Paraffin- und Silikonöle. Zu letzteren zählen u. a. Dialkyl- und Alkylarylsiloxane, wie beispielsweise Dimethylpolysiloxan und Methyl-phenylpolysiloxan, sowie deren alkoxylierte und quaternierte Analoga.

### Fettalkohole mit 8 bis 22 C-Atomen

Die eingesetzten Fettalkohole können gesättigt oder ungesättigt und linear oder verzweigt sein. Einsetzbar im Sinne der Erfindung sind beispielsweise Decanol, Octanol, Octenol, Dodecenol, Decenol, Octadienol, Dodecadienol, Decadienol, Oleylalkohol, Erucaalkohol, Ricinolalkohol, Stearylalkohol, Isostearylalkohol, Cetylalkohol, Laurylalkohol, Myristylalkohol, Arachidylalkohol, Caprylalkohol, Caprinalkohol, Linoleylalkohol, Linolenylalkohol und Behenylalkohol, sowie deren Guerbet-Alkohole. Die Fettalkohole werden üblicherweise aus den Estern der Fettsäuren durch Reduktion gewonnen. Erfindungsgemäß einsetzbar sind ebenfalls Fettalkoholschnitte, die durch Reduktion natürlich vorkommender Fette und Öle, wie z. B. Rindertalg, Erdnußöl, Rüböl, Baumwollsaatöl, Sojaöl, Sonnenblumenöl, Palmkernöl, Leinöl, Rizinusöl, Maisöl, Rapsöl, Sesamöl, Kakaobutter und Kokosfett entstehen. Es können aber auch synthetische Alkohole, z. B. die linearen, geradzahligen Fettalkohole der Ziegler-Synthese (Alfole®) oder die teilweise verzweigten Alkohole aus der Oxosynthese (Dobanole®) verwendet werden.

### Proteine und Proteinderivate

Hierzu zählen wasserlösliche Proteine oder wasserlösliche Derivate unlöslicher Proteine, insbesondere Elastin-, Kollagen-, Keratin-, Milcheiweiß-, Sojaprotein-, Seidenprotein-, Haferprotein-, Erbsenprotein-, Mandelprotein- und Weizenproteinhydrolysate, deren Kondensationsprodukte mit Fettsäuren sowie quaternisierte Proteinhydrolysate.

### Vitamine und Vitaminvorstufen

Vitamin und Vitaminvorstufen wie Tocopherole, Vitamin A, Niacinsäure und Niacinsäureamid, weitere Vitamine des B-Komplexes, Vitamin C, Vitamin F und insbesondere Biotin. Weiterhin bevorzugt innerhalb dieser Gruppe von pflegenden Wirkstoffen sind Panthenol, dessen Derivate, insbesondere die Ester und Ether des Panthenols sowie kationisch derivatisierte Panthenole. Einzelne Vertreter sind beispielsweise das Panthenoltriacetat, der Panthenolmonoethylether und dessen Monoacetat sowie kationische Panthenolderivate.

### Pflanzenextrakte oder daraus gewonnene Wirkstoffe

Pflanzenextrakte, die üblicherweise durch Extraktion der gesamten Pflanze, in einzelnen Fällen aber auch ausschließlich aus Blüten und/oder Blättern der Pflanze hergestellt werden, insbesondere Trockenextrakte, können als Pflegekomponente geeignet sein. Hinsichtlich der erfindungsgemäß verwendbaren Pflanzenextrakte wird insbesondere auf die Extrakte hingewiesen, die in der auf Seite 44 der 3. Auflage des Leitfadens zur Inhaltsstoffdeklaration kosmetischer Mittel, herausgegeben vom Industrieverband Körperpflegeund Waschmittel e.V. (IKW), Frankfurt, beginnenden Tabelle aufgeführt sind. Insbesondere sind Extrakte aus Eichenrinden, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel einsetzbar. Besonders bevorzugt sind die Extrakte aus Mandel, Aloe Vera, Kokosnuß, Mango, Aprikose, Limone, Weizen, Kiwi und Melone. Es können in den erfindungsgemäßen Mitteln auch Mischungen aus mehreren verschiedenen Pflanzenextrakten enthalten sein. Als Extraktionsmittel zur Herstellung der genannten Pflanzenextrakte können u. a. Wasser, Alkohole sowie deren Mischungen verwendet werden. Spezielle Wirkstoffe oder Pflanzenextraktkonzentrate lassen sich durch Einengen der Extrakte gewinnen.

### Weitere Pflegekomponenten

- Honigextrakte, die in analoger Weise zu den Pflanzenextrakten gewonnen werden und üblicherweise 1 - 10 Gew.-%, insbesondere 3 - 5 Gew.-%, Aktivsubstanz enthalten.
- Phospholipide, beispielsweise Sojalecithin, Ei-Lecithin und Kephaline
- Vesikelbildende Lipide, wie z. B: Lecithin, Cholesterol, Sitosterol, Ceramide, Cerebroside und Sphingomyeline
- Ggf. Enzyme, z. B. Proteasen, Amylasen, Lipasen, Cellulasen

### Duftstoffe

Duftstoffe sind ein wesentlicher olfaktorischer Bestandteil der tensidhaltigen Zusammensetzung. Sie werden den erfindungsgemäßen Mitteln zugesetzt, um den ästhetischen Eindruck der Produkte zu verbessern und dem Verbraucher neben der Wasch- und Pflegeleistung ein sensorisch "typisches und unverwechselbares" Produkt zur Verfügung zu stellen. Als Parfümöle bzw. Duftstoffe können einzelne Riechstoffverbindungen, z. B. die synthetischen Produkte vom Typ der Ester, Ether, Aldehyde, Ketone, Alkohole und Kohlenwasserstoffe verwendet werden. Riechstoffe vom Typ der Ester sind z. B. Benzylacetat, Phenoxyethylisobutyrat, p-tert.-Butylcyclohexylacetat, Linalylacetat, Dimethylenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z. B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z. B. die Jonone, ∝-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z. B. Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Üblicherweise liegt der Gehalt an Duftstoffen bei 1 - 5 Gew.-% der Zusammensetzung.

### Überprüfung der inhibitorischen Wirkung

Die hemmende Wirkung der Protease-Inhibitoren wurde sowohl *in vitro* an geeigneten Modellenzymen als auch an Biopsien menschlicher Haut nachgewiesen.

### 1. Inhibitorische Wirkung auf eine Serin-Protease (in vitro)

Da die hauteigenen, am Desmosomenabbau beteiligten Proteasen nicht in ausreichender Menge zur Verfügung standen, dienten die Serin-Proteasen Trypsin und Chymotrypsin (Sigma) aus Rinderpankreas als Modellenzym, um die Hemmwirkung des erfindungsgemäß verwendeten Inhibitors N-CBZ-Gly-Pro-Phe-Pro-Leu, zu testen. Als Vergleich wurde auch das als t-PA-Inhibitor (*t*issue-type *p*lasminogen *a*ctivator) bekannte t-AMCHA [*trans*-4(Aminomethyl)-cyclohexancarbonsäure] getestet.

### Durchführung:

Der Nachweis der Chymotrypsin-Enzymaktivität erfolgt mit einer Modifikation analog zu den Angaben im *Sigma Quality Control Test Procedure*-Datenblatt für Chymotrypsin. Bei Ansätzen ohne Inhibitor wurden 1,42 mL Reagens A (80 mM Tris/HCl-Puffer, pH = 7,8; 25°C) zum Testansatz zugegeben. Bei Ansätzen mit Inhibitor wurden 1,32 mL Reagens A zum Testansatz zugegeben sowie 0,1 mL einer Lösung des entsprechenden Inhibitors in Reagens A (Konzentrationsreihe). Die Enzymaktivität wurde durch Umsetzung des Substrates N-Benzoyl-L-Tyrosinethylester (BTEE) zu N-Benzoyl-L-Tyrosin und Ethanol spektrophotometrisch bestimmt. Die Absorption des proteolytisch abgespaltenen N-Benzoyl-L-Tyrosins wird bei 256 nm gemessen.

Auch der Nachweis der Trypsin-Enzymaktivität erfolgt mit einer Modifikation analog zu den Angaben im *Sigma Quality Control Test Procedure*-Datenblatt für Trypsin. Anstelle von Aprotinin (Reagens F) wurden hier die erfindungsgemäßen Inhibitoren eingesetzt. Hierfür wurde eine Konzentrationsreihe der Inhibitoren in Reagens E angesetzt. Die Enzymaktivität wurde durch Umsetzung des Substrates Nα-Benzoyl-DL-Arginin-p-Nitroanilin (BAPNA) zu Nα-Benzoyl-DL-Arginin und p-Nitroanilin spektrophotometrisch bestimmt. Die Absorption des proteolytisch abgespaltenen p-Nitroanilins wird bei 405 nm gemessen.

Die Reaktionskinetiken wurden je 5 Minuten bei 25 °C detektiert, wobei der lineare Anstieg der Absorption (A) pro Zeiteinheit (t) ein Maß für die Aktivität des Enzyms (ΔA/Δt) ist. Die Aktivität des Enzyms in Abwesenheit eines Proteinase-Inhibitors, (ΔA₁/Δt₁ ), wurde auf 100% gesetzt. Unter analogen Bedingungen wurden die Aktivitäten in Gegenwart eines Inhibitors (ΔA₂/Δt₂) bestimmt. Die Hemmwirkung oder Minderung der Enzymaktivität entspricht dann: 100% - (ΔA₂/Δt₂ )/( ΔA₁/Δt₁)%.

Die Beispiele 1-3, 5-10, 12-14 fallen nicht unter die Ansprüche.

### Hemmwirkung auf die Trypsin- bzw. Chymotrypsin-Aktiviät

### Beispiel 1: Phenylboronsäure als Inhibitor im Enzymtest

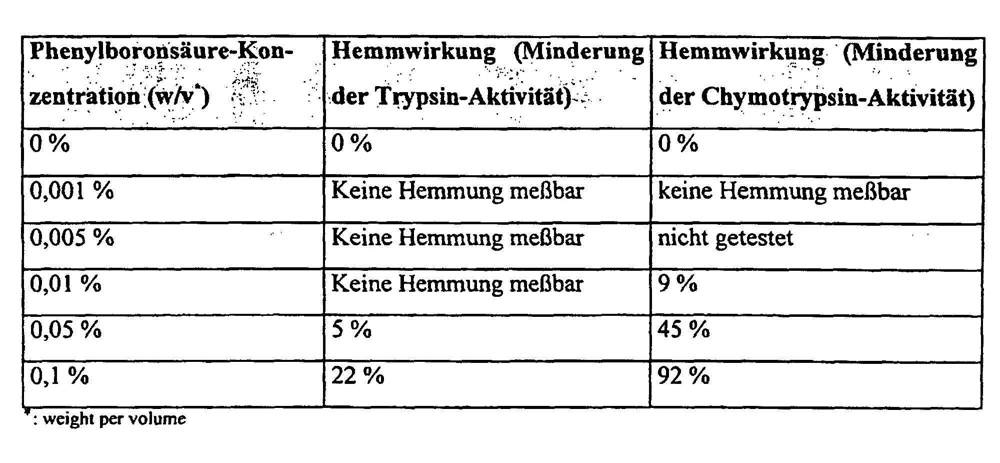

Durch Phenylboronsäure wird die Aktivität von Trypsin im gewählten Konzentrationsbereich nur mäßig beeinträchtigt. Hingegen wird eine deutliche, konzentrationsabhängige Hemmung von Chymotrypsin beobachtet.

### Beispiel 2: Borsäure als Inhibitor im Enzymtest

| **Borsäure-Konzentration (w/v*)** | **Hemmwirkung (Minderung der Trypsin-Aktivität)** | **Hemmwirkung (Minderung der Chymotrypsin-Aktivität)** |
|---|---|---|
| 0% | 0% | 0% |
| 0,001 % | Keine Hemmwirkung meßbar | keine Hemmwirkung meßbar |
| 0,01 % | Keine Hemmwirkung meßbar | keine Hemmwirkung meßbar |
| 0,1% | 6 % | 23 % |
| 0,5 % | 23 % | nicht getestet |

| | | |
|---|---|---|
| *: weight per volume | | |

Die Hemmwirkung von Borsäure auf Trypsin und Chymotrypsin ist im getesteten Konzentrationsbereich nur schwach. Chymotrypsin wird bei geringerer Borsäure-Konzentration stärker gehemmt als Trypsin.

### Beispiel 3: 4-(2-Aminoethyl)phenylsulfonylfluorid (Pefabloc® SC) als Inhibitor im Enzymtets

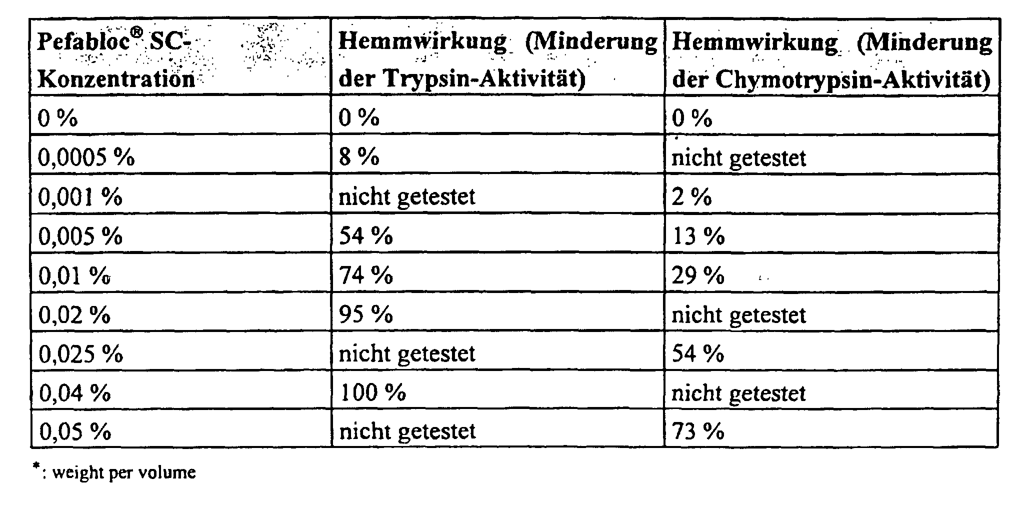

Pefabloc® SC zeigt im getesteten Konzentrationsbereich eine starke, konzentrationsabhängige Hemmung von Trypsin und Chymotrypsin.

### Beispiel 4: Pentapeptidsequenz GPFPL als Inhibitor im Enzymtest

| **N-CBZ-GPFPL-Konzentration (w/v*)** | **Hemmwirkung (Minderung der Trypsin-Aktivität)** | **Hemmwirkung (Minderung der Chymotrypsin-Aktivität)** |
|---|---|---|
| 0,03 % | keine Hemmwirkung messbar | 2 % |
| 0,06 % | -"- | 7 % |
| 0,33 % | -"- | 27 % |

| | | |
|---|---|---|
| *: weight per volume | | |

Die Trypsin-Aktivität wird durch GPFPL im gewählten Konzentrationsbereich nicht beeinträchtigt. Bei einer Konzentration des Inhibitors von 0,33 Gew.% wird eine spezifische Hemmung (27%) von Chymotrypsin nachgewiesen.

### Beispiel 5: Rosmarinsäure als Inhibitor im Enzymtest

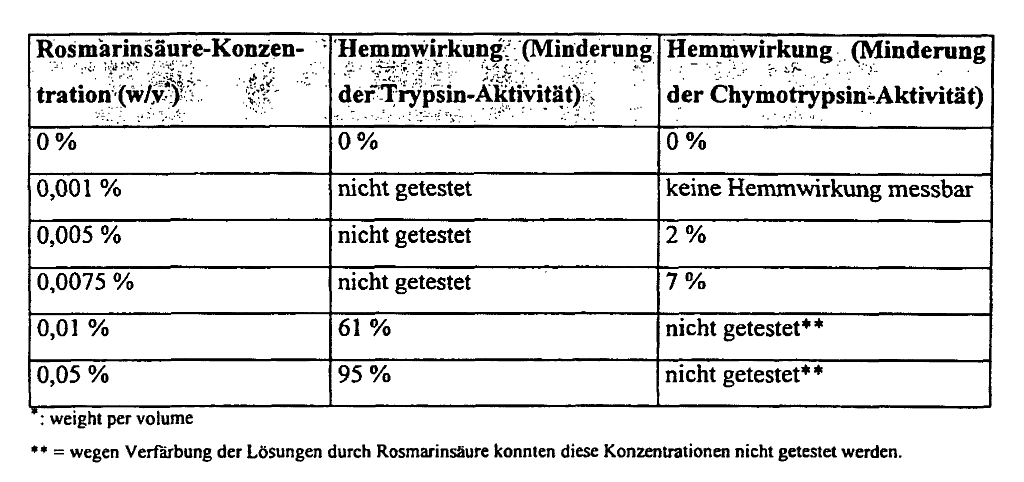

Rosmarinsäure zeigt eine starke, konzentrationsabhängige Hemmung von Trypsin. Die Hemmung von Chymotrypsin ist bei den untersuchbaren, niedrigen Inhibitor-Konzentrationen gering.

### Beispiel 6: Elhibin® als Inhibitor im Enzymtest

| **Elhibin®-Konzentration (v/v*)** | **Hemmwirkung (Minderung der Trypsin-Aktivität)** | **Hemmwirkung (Minderung der Chymotrypsin-Aktivität)** |
|---|---|---|
| 0% | 0% | 0% |
| 0,01 % | 8% | 7% |
| 0,05 % | 39 % | 30 % |
| 0,1 % | 76 % | 55 % |
| 0,5 % | 98 % | 92 % |
| 1,0 % | nicht getestet | 97 % |

| | | |
|---|---|---|
| *: volume per volume | | |

Sowohl Trypsin als auch Chymotrypsin werden durch Elhibin® gleichermaßen stark im getesteten Konzentrationsbereich gehemmt.

### Beispiel 7: t-AMCHA als Inhibitor im Enzymtest

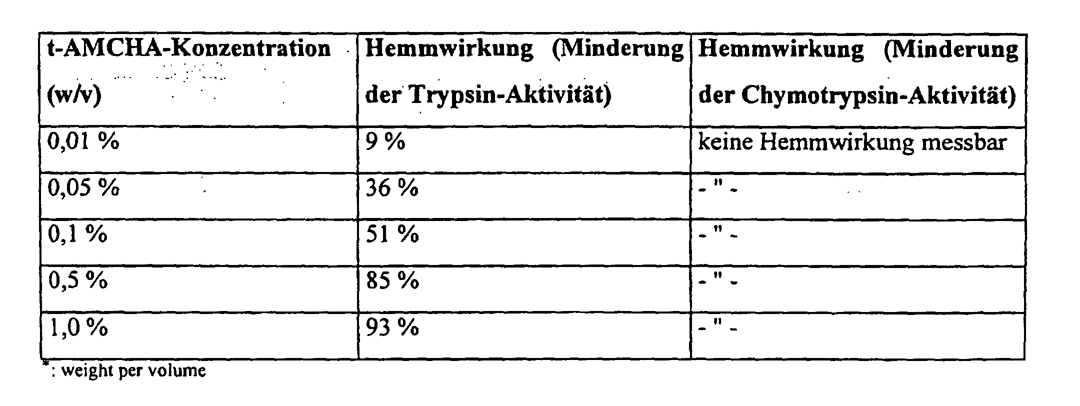

Nur die Trypsin-Aktivität wird durch t-AMCHA in Abhängigkeit von der Inhibitor-Konzentration beeinträchtigt. Hingegen wird die Aktivität von Chymotrypsin im gleichen Konzentrationsbereich durch t-AMCHA nicht beeinträchtigt.

### Bewertung der Tests:

Die Testergebnisse zeigen, daß der Inhibitor t-AMCHA (Beispiel 7) im untersuchten Konzentrationsbereich nur Trypsin hemmt und sich somit von den anderen verwendeten Inhibitoren unterscheidet. Einige der getesteten Inhibitoren erzielen bei sehr geringer Konzentration eine effektive Hemmung.

Eine spezifische Hemmung der Chymotrypsin-Aktivität wurde für niedrige Konzentrationen des Pentapeptids GPFPL beobachtet. Borsäure zeigt im getesteten Konzentrationsbereich eine schwache Hemmung beider Enzyme, Phenylboronsäure zeigt bereits in geringer Konzentration (0,1 %) eine schwache Hemmung von Trypsin und eine starke Hemmung von Chymotrypsin. Eine nahezu vollständige Hemmung beider Enzyme tritt auch mit Elhibin® (0,5 %) ein. Geringe Konzentrationen Rosmarinsäure (0,05 %) sind ein äußerst effektiver Inhibitor für Trypsin. Als effektiver Inhibitor beider Enzyme hat sich auch Pefabloc® erwiesen, wobei im untersuchten Konzentrationsbereich Trypsin stärker gehemmt wird als Chymotrypsin.

### 2. Tests der Inhibitoren an Haut-Biopsien

Der Einfluß der Protease-Inhibitoren Phenylboronsäure, Borsäure, des Pentapeptids N-CBZ-Gly-Pro-Phe-Pro-Leu, Rosmarinsäure und Elhibin® auf die Desquamation des Stratum corneum wurde an Haut-Biopsien untersucht.

Aus frischen Humanhautexplantaten (aus Mammareduktion) wurden 8mm²-große Hautstücke ausgestanzt und mit einer Mischung aus Detergentien (Na-Dodecylsulfat, N,N-Dimethyldodecylaminoxid) in Abwesenheit und in Gegenwart der Inhibitoren behandelt. Unter dem Einfluß der Detergentien wird eine verstärkte Abschuppung (Desquamation) der Homschicht (Stratum corneum) künstlich induziert, also die Ausbildung trockener Haut im Versuch nachgeahmt. Die Anzahl abgeschuppter Hornzellen (Korneozyten) wurde unter dem Mikroskop in einer Schilling-Zählkammer bestimmt. Eine Verringerung der Korneozytenzahl weist auf eine hemmende Wirkung der getesteten potentiellen Inhibitoren hin.

### Durchführung:

Die Inhibitoren wurden in den entsprechenden Konzentrationen (vgl. Tabellen der Beispiele 7-12) im Inkubationspuffer angesetzt. Dieser bestand aus 0,1 M TRIS-HCl-Puffer (pH 8) mit 0,1 Gew.% Na-Azid und den Detergenzien Na-Dodecylsulfat (0,2 mM) und N,N-Dimethyldodecylaminoxid (8 mM) sowie EDTA (5 mM). Diese Inkubationspufferlösungen mit variierender Konzentration des Inhibitors werden nachfolgend als Testlösungen bezeichnet.

Es wurden zwei Arten von Blindkontrollen durchgeführt, eine Positiv- und eine Negativkontrolle. Für die sogenannte *Positivkontrolle* wurde nur der Inkubationspuffer verwendet, d. h. ohne Zusatz der Inhibitoren. Für die sogenannte *Negativkontrolle* wurde eine 5 mM Lösung von EDTA in TRIS-HCl-Puffer eingesetzt, d. h. ohne Zusatz der Detergentien.

Pro Reaktionsansatz wurden 0,5 mL der jeweiligen Testlösung oder des für die Blindkontrollen verwendeten Puffergemisches in verschließbaren Reaktionsgefäßen vorgelegt. Alle Ansätze wurden in vierfacher Ausfertigung durchgeführt, um die Reproduzierbarkeit der Ergebnisse zu gewährleisten.

Die Humanhaut wurde nach der Explantation in einer trockenen, sterilen Petrischale auf Kühlakkus transportiert. Nach Entfernung des Unterhautfett- und des Bindegewebes wurden mit Hilfe von sterilen Stanzen 8 mm²-große Hautstücke präpariert. Diese wurden 15-20 min in steriler, physiologischer NaCl (0,9 %) gewaschen, zu den Reaktionsansätzen gegeben (1 Hautstück/Reaktionsansatz) und 44 Stunden bei 37° C inkubiert.

Nach Beendigung der Inkubationsphase wurden die Reaktionsgefäße 30 Sekunden auf einem Vortex-Mixer geschüttelt, um noch lose anhaftende Komeozyten von den Hautstücken abzulösen. Die Hautstücke wurden entnommen und die verbleibende Dispersion wurde 8 Minuten bei 11.000U/min zentrifugiert, um die Korneozyten zu isolieren. Der detergenshaltige Überstand (0,4 mL) wurde verworfen und der Rückstand mit 0,4 ml Aqua dest. gewaschen. Nach nochmaligem Zentrifugieren wurde der Überstand (0.4 mL) wiederum verworfen, das verbleibende Zellpellet in 0,4 mL Aqua dest. aufgenommen und die abgelösten Korneozyten in einer Schillingzählkammer unter einem Lichtmikroskop ausgezählt.

Nachfolgende Tabellen geben eine Zusammenstellung über die Anzahl abgelöster Korneozyten:
- nach Behandlung der Haut-Biopsien mit einem Detergentiengemisch in Abwesenheit der Inhibitoren (Positivkontrolle)
- nach Behandlung der Haut-Biopsien mit einer Pufferlösung in Abwesenheit der Detergentien und Inhibitoren (Negativkontrolle)
- nach Behandlung der Haut-Biopsien mit einem Detergentiengemisch, welches die Inhibitoren in variierender Konzentration enthält.

### Beispiel 8: Phenylboronsäure als Inhibitor im Desquamationstest

Phenylboronsäure wurde in verschiedenen Konzentrationen als Desquamationsinhibitor getestet. Der Korneozytenwert der Positivkontrolle wurde auf 100 % gesetzt.

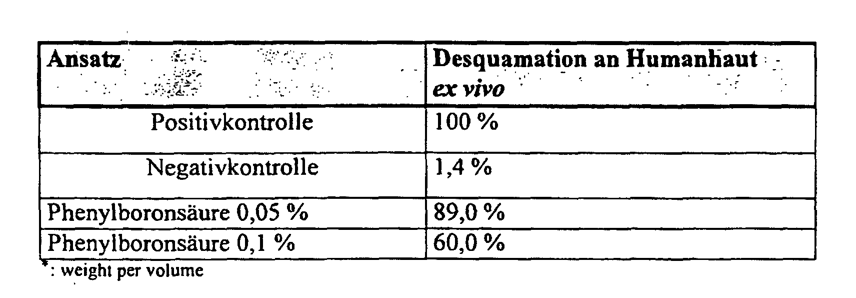

In einer Konzentration von 0,1 % konnte Phenylboronsäure die Abschuppung der Korneozyten siginifikant reduzieren.

### Beispiel 9: Borsäure als Inhibitor im Desquamationstest

Borsäure wurde in verschiedenen Konzentrationen als Desquamationsinhibitor getestet. Der Komeozytenwert der Positivkontrolle wurde auf 100 % gesetzt.

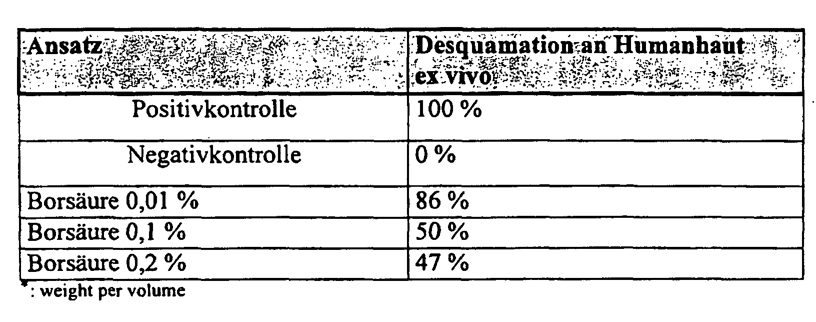

In einer Konzentration von 0,2 % konnte Borsäure die Abschuppung der Korneozyten zu halbieren.

### Beispiel 10: Pefabloc® SC als Inhibitor im Desquamationstest

4-(2-Aminoethyl)phenylsulfonylfluorid (Pefabloc® SC) wurde in verschiedenen Konzentrationen als Desquamationsinhibitor getestet. Der Korneozytenwert der Positivkontrolle wurde auf 100 % gesetzt.

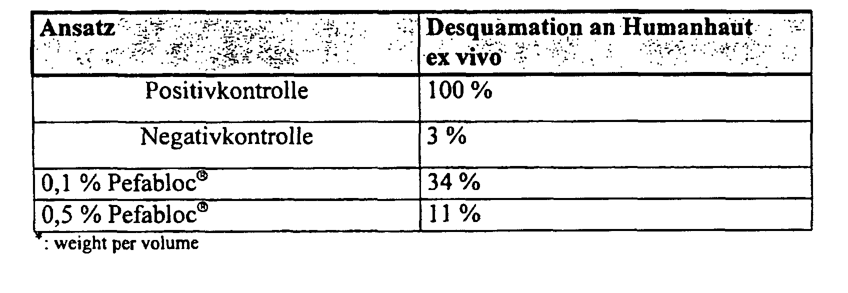

Bei Verwendung von 0,5 % Pefabloc® SC wurde die Abschuppung um nahezu 90 % reduziert.

### Beispiel 11: Z-GPFPL als Inhibitor im Desquamationstest

Das Pentapeptid N-CBZ-Gly-Pro-Phe-Pro-Leu (GPFPL) wurde in verschiedenen Konzentrationen als Desquamationsinhibitor getestet. Der Komeozytenwert der Positivkontrolle wurde auf 100 % gesetzt.

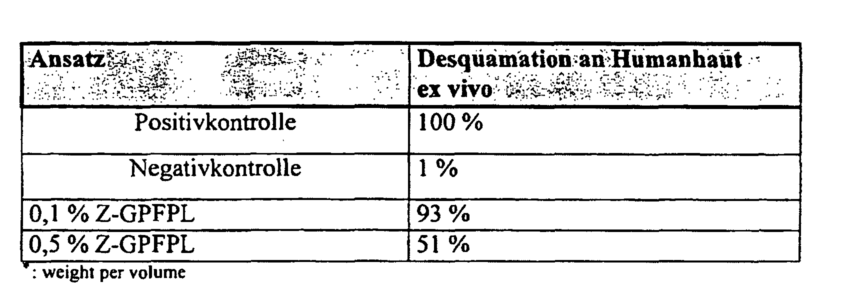

Bei Verwendung von 0,5 % des Pentapetids wurde die Abschuppung um die Hälfte reduziert.

### Beispiel 12: Rosmarinsäure als Inhibitor im Desquamationstest

Rosmarinsäure wurde in verschiedenen Konzentrationen als Desquamationsinhibitor getestet. Der Korneozytenwert der Positivkontrolle wurde auf 100 % gesetzt.

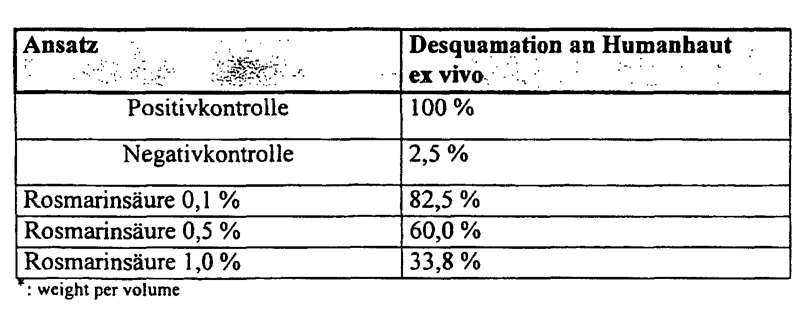

Auch Rosmarinsäure bewirkte eine konzentrationsabhängige Reduktion der Korneozytenabschuppung. Bei Verwendung von 1,0 % Rosmarinsäure kann die Abschuppung auf 33,8 % reduziert werden.

### Beispiel 13: Elhibin® als Inhibitor im Desquamationstest

Elhibin® wurde in verschiedenen Konzentrationen als Desquamationsinhibitor getestet. Der Komeozytenwert der Positivkontrolle wurde auf 100 % gesetzt.

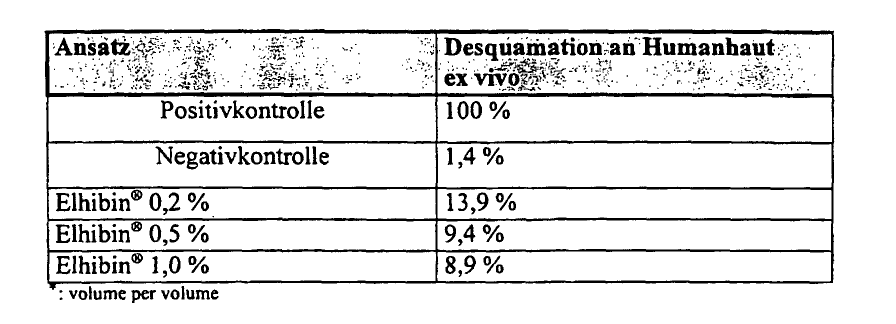

Elhibin® wirkt auch in geringer Konzentration als starker Desquamationsinhibitor und zeigt bezüglich der Korneozytenabschuppung im untersuchten Konzentrationsbereich ein schwach konzentrationsabhängiges Profil.

### Beispiel 14: t-AMCHA als Inhibitor im Desquamationstest

Als Inhibitor wurde auch t-AMCHA eingesetzt. Der Korneozytenwert der Positiv-Kontrolle wurde auf 100 % gesetzt.

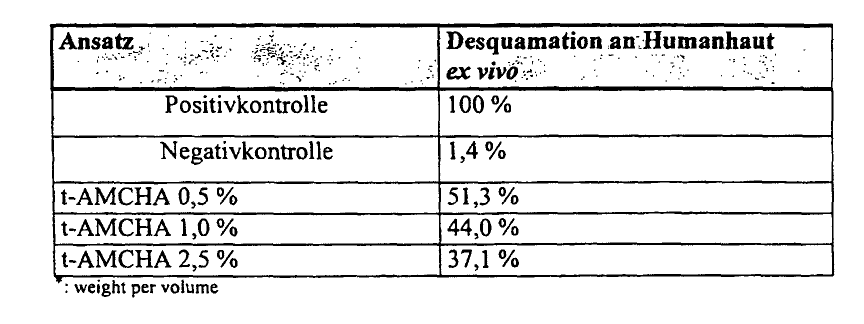

Auch t-AMCHA erwies sich als Desquamationsinhibitor mit konzentrationsabhängigem Profil. Allerdings ist die t-AMCHA-Konzentration vergleichsweise höher, um eine entsprechende Reduktion der Desquamation zu erreichen.

### Bewertung der Tests:

Die durch die Detergenzienmischung verursachte Desquamation der Humanhaut kann durch Phenylboronsäure, Borsäure, das Pentapeptid Z-Gly-Pro-Phe-Pro-Leu, Rosmarinsäure, Elhibin® und t-AMCHA signifikant reduziert werden. Dabei zeigten sich konzentrationsabhängige Effekte, d. h. mit Zunahme der Inhibitorkonzentration nimmt die Korneozyten-Abschuppung ab.

### Nicht unter die Ansprüche fallende Formulierungsbeispiele

Alle nachfolgenden Mengenangaben beziehen sich auf Gew.-% der handelsüblichen Substanzen oder Substanzgemische bezogen auf die Gesamtmenge der Zusammensetzung.
(EO = angelagerte Mol Ethylenoxid).

### 1. Duschgel

7,0 % Natriumlaurethsulfat (z. B. Texapon® N70)
5,0 % Natrium-PEG-6-cocamidcarboxylat (z. B. Akypo-Soft® KA 250 BVC)
4,0 % Laurylglucosid (z. B. Plantacare® 1200 UP)
6,0 % PEG-7-Glycerylcocoat (z. B. Cetiol® HE)
0,2 % Ricinusölpolyglycolether, gehärtet (z. B. Eumulgin® HRE 455)
8,0 % Laureth-2 (z. B. Arlypon® F)
1,0 % Isopropylmyristat
1,0 % Parfümöl
0,08 % Polyquaternium-7 (z. B. Conditioner® P7)
1,0 % Natriumchlorid
0,25 % Citronensäure
0,4 % Natriumbenzoat
0,2 % Salicylsäure
4,0 % Elhibin® oder 1 % Pefabloc® SC
ad 100% H₂O (dest.)

Isopropylmyristat, Parfümöl, Eumulgin® HRE 455 und Cetiol® HE wurden gemischt. Die übrigen Komponenten wurden der Reihe nach in Wasser gelöst. Schließlich wurde die Mischung der Ölkomponenten und die wasserfreien Tenside in die wäßrige Lösung eingearbeitet. Zuletzt wurde das Arlypon F zugesetzt und durch Na-Cl die Viskosität eingestellt.

### 2. Schaumbad

15,0 % Natriumlaurethsulfat (z. B. Texapon® N70)
7,0 % Laurylglucosid (z. B. Plantacare® 1200 UP)
3,0 % N,N-Dimetyl-N(kokosamidopropyl)ammoniumacetobetain (z. B. Dehyton® PK 45)
0,2 % Ricinusölpolyglycolether, gehärtet (z. B. Eumulgin® HRE 455)
1,0 % PEG-7-Glycerylcocoat (z. B. Cetiol® HE )
1,0 % Glycerin
0,5 % Milchsäure
0,08 % Polyquatemium-7 (z. B. Conditioner® P7)
0,4 % Natriumbenzoat
3,0 % 4-(2-Aminoethyl)phenylsulfonylfluorid (z. B. Pefabloc® SC)
0,9 % Natriumchlorid
1,0 % Parfümöl
0,2 % Citronensäure
2,0 % Perlglanzmittel (z. B. Euperlan® PK 810)
qs. Farbstoffe
ad 100% H₂O (dest.)

### 3. Anti-Schuppen-Shampoo

15,0 % Natriumlaurethsulfat (z. B. Texapon® N70)
7 % Dinatriumcocamphodiacetat (z.B. Rewoteric® AM 2C/NM)
3 % Perlglanzmittel (z. B. Euperlan® PK 810)
0,5 % Polyquaternium-10 (Z. B. Polymer JR® 400)
2,0 % Elhibin®
0,2 % Konservierungsmittel
qs. Citronensäure
qs. Natriumchlorid
ad 100 % Wasser

### 4. Handgeschirrspülmittel-Konzentrat (Rahmenrezeptur)

25,0 % Natriumlaurethsulfat (z.B. Texapon® NSO)
3,0 % C₁₂₋₁₆-Alkylpolyglucosid (z. B. Plantacare® 1200 UP)
2,0 % Cocamidopropylbetain (z. B. Tego-Betain® BL-215)
8,0 % Ethanol (96%-ig)
3,0 % Polycarboxylat, Na-Salz
0,5 % Citronensäure
0,4 % Konservierungsmittel
0,3 % Parfüm
3,0 % Rosmarinsäure
ad 100% H₂O (dest.)

### 5. Reiniger (Rahmenrezeptur)

2,0 % Natriumlaurethsulfat (z.B. Texapon® NSO)
10,0 % C₁₂₋₁₆-Alkylpolyglucosid (z. B. Plantacare® 1200 UP)
1,0 % Cocamidopropylbetain (z. B. Tego-Betain® BL-215)
5,0 % Ethanol (96%-ig)
1,5 % Natriumchlorid
3,0 % Citronensäure
3,0 % Harnstoff
0,4 % Konservierungsmittel
2,0 % Parfüm
4,0 % Rosmarinsäure
ad 100% H₂O (dest.)

### Anhang

### Verzeichnis der Warenzeichen der verwendeten Rohstoffe

1) Tego-Betain® BL-215
   INCI: Cocamidopropyl betaine
   Hersteller: Goldschmidt
2) Texapon® NSO
   INCI: Sodium laureth sulfate
   Hersteller: Cognis Deutschland GmbH (Henkel)
3) Dehyton® PK 45
   INCI: Aqua (water), Cocamido propyl betaine
   Hersteller: Goodrich
4) Euperlan® PK 810
   INCI: Aqua (Wasser), Glycol distearate, Sodium laureth sulfate, Cocamide MEA, Laureth-10, Formic acid
   Hersteller: Cognis Deutschland GmbH (Henkel)
5) Rewoteric® AM2C/NM
   INCI: Disodium cocamphodiacetate
   Hersteller: CK Witco (Rewo)
6) Polymer JR® 400
   INCI: Polyquaternium-10
   Hersteller: National Starch
7) Eumulgin® HRE 455
   INCI: PEG-40-hydrogenated castor oil, propylene glycol, aqua (water)
   Hersteller: Cognis Deutschland GmbH (Henkel)
8) Cetiol® HE
   INCI: PEG-7-Glyceryl cocoate
   Hersteller: Cognis Deutschland GmbH (Henkel)
9) Pefabloc®
   4-(2-Aminoethyl)phenylsulfonylfluorid
   Hersteller: Boehringer Mannheim
10) Pefabloc® SC
   4-(2-Aminoethyl)phenylsulfonylfluorid mit speziellem Protector
   Hersteller: Boehringer Mannheim
11) Texapon® N 70
   Natriumlaurethsulfat
   Hersteller: Cognis Deutschland GmbH (Henkel)
12) Akypo-Soft® KA 250 BVC
   INCI: Sodium-PEG-6-cocamide carboxylate
   Hersteller: Kao Chemicals
13) Plantacare® 1200 UP
   INCI: Lauryl glucoside
   Hersteller: Cognis Deutschland GmbH (Henkel)
14) Arlypon® F
   INCI: Laureth-2
   Hersteller: Cognis Deutschland GmbH (Henkel)
15) Conditioner® P7
   INCI: Polyquaternium-7
   Hersteller: Sigma, Bergamo (Sivax)

## Patentansprüche

1. Tensidhaltige Reinigungsmittel mit einem Tensidgehalt von wenigstens 5 Gew.-%, **dadurch gekennzeichnet, dass** mindestens eine als Inhibitor für die am Desmosomen-Abbau beteiligten Proteinasen wirkende Substanz, die in einer Konzentration von wenigstens 0,0005 Gew.-% eine wenigstens 5%-ige Hemmung erzeugt, ausgewählt aus Verbindungen, enthaltend die Pentapeptidsequenz Glycin-Prolin-Phenylalanin-Prolin-Leucin, enthalten ist.

2. Mittel gemäß Anspruch 1, **dadurch gekennzeichnet, dass** die Verbindungen, enthaltend die Pentapeptidsequenz Glycin-Prolin-Phenylalanin-Prolin-Leucin, ausgewählt sind aus endgruppengeschützten Derivaten dieser Pentapeptidsequenz, Polypeptiden, Proteinen und anderen chemischen Derivaten, die diese Pentapeptidsequenz enthalten.

3. Mittel gemäß Anspruch 1, **dadurch gekennzeichnet, dass** die Verbindung, enthaltend die Pentapeptidsequenz Glycin-Prolin-Phenylalanin-Prolin-Leucin, N-Benzyloxycarbonyl-Gly-Pro-Phe-Pro-Leu ist.

4. Mittel gemäß einem der Ansprüche 1 - 3, **dadurch gekennzeichnet, dass** die Proteinase-Inhibitoren in einer Konzentration von 0,1-20 Gew.-% in der Zusammensetzung enthalten sind.

5. Verwendung von Verbindungen, enthaltend die Pentapeptidsequenz Glycin-Prolin-Phenylalanin-Prolin-Leucin, zur Herstellung von tensidhaltigen Reinigungsmitteln mit einem Tensidgehalt von wenigstens 5 Gew.-% als Inhibitoren für die am Desmosomen-Abbau beteiligten Proteinasen, die in einer Konzentration von wenigstens 0,0005 Gew.-% eine wenigstens 5%-ige Hemmung erzeugen, zur Verhinderung oder Verminderung der Hautaustrocknung.

6. Verwendung gemäß Anspruch 5, **dadurch gekennzeichnet, dass** die Verbindungen, enthaltend die Pentapeptidsequenz Glycin-Prolin-Phenylalanin-Prolin-Leucin, in einer Konzentration von 0,1-20 Gew.-% in dem tensidhaltigen Reinigungsmittel enthalten sind.

7. Verfahren zur manuellen Reinigung harter Oberflächen mit tensidhaltigen Reinigungsmitteln bei gleichzeitiger Verhinderung oder Verminderung der Hautaustrocknung, **dadurch gekennzeichnet, dass** man die Oberflächen mit einem tensidhaltigen Reinigungsmittel mit einem Tensidgehalt von wenigstens 5 Gew.-%, enthaltend 0,1 - 20 Gew.-% Gew.-% mindestens eines Proteinase-Inhibitors, der in einer Konzentration von wenigstens 0,0005 Gew.-% eine wenigstens 5%-ige Hemmung erzeugt und der ausgewählt ist aus Verbindungen, enthaltend die Pentapeptidsequenz Glycin-Prolin-Phenylalanin-Prolin-Leucin, behandelt.

## Claims

1. A surfactant-containing cleaning composition with a surfactant content of at least 5% by weight, **characterized in that** it contains at least one substance acting as an inhibitor for the proteinases involved in desmosome degradation, which produces an at least 5% inhibition in a concentration of at least 0.0005% by weight, selected from compounds containing the pentapeptide sequence glycine-proline-phenyl alanine-proline-leucine.

2. A cleaning composition as claimed in claim 1, **characterized in that** the compounds containing the pentapeptide sequence glycine-proline-phenyl alanine-proline-leucine are selected from terminal-group-protected derivatives of this pentapeptide sequence, polypeptides, proteins and other chemical derivatives containing this pentapeptide sequence.

3. A cleaning composition as claimed in claim 1, **characterized in that** the compound containing the pentapeptide sequence glycine-proline-phenyl alanine-proline-leucine is N-benzyloxycarbonyl-gly-pro-phe-pro-leu.

4. A cleaning composition as claimed in any of claims 1 to 3, **characterized in that** the proteinase inhibitors are present in the composition in a concentration of 0.1 to 20% by weight.

5. The use of compounds containing the pentapeptide sequence glycine-proline-phenyl alanine-proline-leucine for the production of surfactant-containing cleaning compositions with a surfactant content of at least 5% by weight as inhibitors for the proteinases involved in desmosome degradation, which produce an at least 5% inhibition in a concentration of at least 0.0005% by weight, for preventing or reducing drying out of the skin.

6. The use claimed in claim 5, **characterized in that** the compounds containing the pentapeptide sequence glycine-proline-phenyl alanine-proline-leucine are present in the surfactant-containing cleaning composition in a concentration of 0.1 to 20% by weight.

7. A process for the manual cleaning of hard surfaces with surfactant-containing cleaning compositions in which drying out of the skin is prevented or reduced, **characterized in that** the surfaces are treated with a surfactant-containing cleaning composition having a surfactant content of at least 5% by weight and containing 0.1 to 20% by weight of at least one proteinase inhibitor which produces an at least 5% inhibition in a concentration of at least 0.0005% by weight and which is selected from compounds containing the pentapeptide sequence glycine-proline-phenyl alanine-proline-leucine.

## Revendications

1. Agent de nettoyage contenant un ou des agents tensioactifs, présentant une teneur en agent(s) tensioactif(s) d'au moins 5% en poids, **caractérisé en ce qu'**il contient au moins une substance agissant comme inhibiteur pour les protéinases participant à la dégradation des desmosomes, qui produit une inhibition d'au moins 5% en une concentration d'au moins 0,0005% en poids, choisie parmi les composés qui contiennent la séquence pentapeptidique glycine-proline-phénylalanine-proline-leucine.

2. Agent selon la revendication 1, **caractérisé en ce que** les composés, contenant la séquence pentapeptidique glycine-proline-phénylalanine-proline-leucine, sont choisis parmi les dérivés à groupes terminaux protégés de cette séquence pentapeptidique, les polypeptides, les protéines et les autres dérivés chimiques, qui contiennent cette séquence pentapeptidique.

3. Agent selon la revendication 1, **caractérisé en ce que** le composé, contenant la séquence pentapeptidique glycine-proline-phénylalanine-proline-leucine, est le N-benzyloxycarbonyl-Gly-Pro-Phe-Pro-Leu.

4. Agent selon l'une quelconque des revendications 1 à 3, **caractérisé en ce que** les inhibiteurs de protéinase sont contenus dans la composition en une concentration de 0,1-20% en poids.

5. Utilisation de composés contenant la séquence pentapeptidique glycine-proline-phénylalanine-proline-leucine pour la préparation d'agents de nettoyage contenant un ou des agents tensioactifs, présentant une teneur en agent(s) tensioactif(s) d'au moins 5% en poids comme inhibiteurs de protéinases participant à la destruction de desmosomes, qui produisent une inhibition d'au moins 5% en une concentration d'au moins 0,0005% en poids pour éviter ou diminuer le séchage de la peau.

6. Utilisation selon la revendication 5, **caractérisée en ce que** les composés, contenant la séquence pentapeptidique glycine-profine-phénylatanine-proline-leucine, sont contenus dans l'agent de nettoyage contenant un ou des agents tensioactifs en une concentration de 0,1-20% en poids.

7. Procédé pour le nettoyage manuel de surfaces dures avec des agents de nettoyage contenant un ou des agents tensioactifs en évitant ou en diminuant simultanément le séchage de la peau, **caractérisé en ce qu'**on traite les surfaces avec un agent de nettoyage contenant un ou des agents tensioactifs présentant une teneur en agent(s) tensioactif(s) d'au moins 5% en poids, contenant 0,1 à 20% en poids d'au moins un inhibiteur de protéinase qui produit une inhibition d'au moins 5% en une concentration d'au moins 0,0005% en poids et qui est choisi parmi les composés contenant la séquence pentapeptidique glycine-proline-phénylalanine-proline-leucine.
